# EUROPEAN PATENT SPECIFICATION

(11) **EP 0 625 904 B1**
(45) Date of publication and mention of the grant of the patent: **28.05.2003**
(21) Application number: 93905811.1
(22) Date of filing: 09.02.1993
(51) Int. Cl.: A61K 31/715, A61P 27/02

(54) **BIMODAL MOLECULAR WEIGHT HYALURONATE FORMULATIONS AND METHODS FOR USING SAME**
ZUSAMMENSETZUNGEN VON HYALURONAT MIT BIMODALEM MOLEKULARGEWICHT UND VERFAHREN ZU DEREN VERWENDUNG
FORMULATIONS A BASE D'HYALURONATE A POIDS MOLECULAIRE BIMODAL ET PROCEDES D'UTILISATION

(30) Priority: 10.02.1992 US 832972
(43) Date of publication of application: 30.11.1994
(73) Proprietor: Advanced Medical Optics, Inc., Santa Ana, CA 92705 (US)
(72) Inventor: CHRIST, F., Richard, Laguna Beach, CA 92651 (US)
(74) Representative: Hutchins, Michael Richard
(86) International application number: US9301088
(87) International publication number: WO93015744

(56) References cited:
- EP-A- 0 138 572
- EP-A- 0 197 718
- FR-A- 2 478 468
- PATENT ABSTRACTS OF JAPAN vol. 13, no. 487 (C-649)1989
- BULL. MEM. SOC. FR. OPHTHALMOL. vol. 97, 1986, pages 556 - 559 L. TAVERNITI 'Hyaluronate de sodium intracamérulaire et altérations de la barrière hémato-oculaire.'
- ANN. OTTALMOL. CLIN. OCUL. vol. 115, no. 6, 1989, pages 567 - 571 V. MARCHI 'Vantaggi derivanti dall'uso dello hyalectin in casi speciali di microchirurgia oftalmica: Ciclodiastasi, chirurgia refrattiva, cheratoprotesi.'

## Description

### Related Application

This application is a continuation-in-part of copending application Serial No. 621,290 filed November 30, 1990.

### Background of the Invention

This invention relates to compositions and methods useful to protect human or animal eye cell layers and tissue subject to exposure to trauma. More particularly, the invention relates to compositions and methods involving hyaluronate fractions having differing molecular weights for protecting eye cell layers and tissues exposed to trauma, for example, during surgery.

When the natural lens of the eye becomes hazy or clouded, surgery is often indicated to remove the impaired lens. The current trend in such surgery is toward breaking the lens into a plurality of particles and then removing the particles, e.g., using the conventional phacoemulsification procedure. The use of a protective agent, in particular a viscoelastic fluid, that adheres to and protects the corneal endothelium during the surgical procedure is highly beneficial. Fluids containing high molecular weight range hyaluronates, that is hyaluronates having weight average molecular weights over 750,000, do not provide entirely acceptable adhesion to and/or protection of the corneal endothelium during certain surgical procedures. An example of such a fluid is that sold by Pharmacia Ophthalmics of Monrovia, California under the trademark Healon.

Formulations which include hyaluronic acid having a molecular weight of at least about 750,000, and preferably at least about 1,200,000 are disclosed in Balazs U.S. Patent 4,141,973. Such formulations, which are not described in this patent as including any lower molecular weight hyaluronic acid, are disclosed as being useful in a number of applications, such as in the replacement of the aqueous humor after various intraocular surgical procedures, and as a biological prosthesis in the anterior chamber after cataract surgery.

Another formulation which includes high molecular weight hyaluronate (molecular weight of 1x10⁶ to 4.5x10⁶) is that disclosed in Balazs U.S. Patent 4,303,672. The cosmetic formulations of this patent also include a low molecular weight hyaluronate fraction (molecular weight of 10,000 to 200,000) and protein in an amount ranging from 50% to 400% the weight of the hyaluronate. Such compositions are taught as having emollient, moisturizing, elasticizing and lubricating properties where applied to the skin. Such protein-containing hyaluronate compositions are not ophthalmically acceptable and would not be useful as protective agents in eye surgery.

Bracke et al U.S. Patent 4,517,295 discloses hyaluronic acid having an average molecular weight of about 55,000 which is produced from bacterial sources. Such hyaluronic acid is disclosed as having potentially significant use as an eye drop ingredient and as an ingredient of cosmetic formulations, as well as being useful in post-surgical applications for reducing complications due to fibrotic response and/or adhesion formation. This patent does not disclose the use of such hyaluronic acid as a protective agent in eye surgery.

Other formulations incorporate combinations of different materials, such as sodium hyaluronate and chondroitin sulfate. This type of product is described in Australian Patent 555,747. A product for use in providing protection during eye surgery and including sodium hyaluronate and chondroitin sulfate is that sold by Alcon Surgical, Inc. of Forth Worth, Texas under the trademark Viscoat. Sources of chondroitin sulfate often include significant amounts of protein which need to be removed, using relatively complex and expensive separation techniques, before the purified chondroitin sulfate can be used in the eye.

There continues to be a need for compositions useful in protecting ocular or eye cell layers and tissues exposed to trauma, in particular trauma involved in ophthalmic surgical procedures.

### Summary of the Invention

New compositions and methods according to claims 1 to 19 useful to protect human or animal ocular or eye cell layers and tissues subject to exposure to trauma have been discovered. The present ophthalmically acceptable compositions, which include two distinct molecular weight fractions of alkali metal and/or alkaline earth metal hyaluronates, have been found to provide a substantial degree of protection against trauma, such as that trauma which occurs during eye surgery, in particular eye surgery such as the removal of a diseased natural lens. The present compositions, which are based on hyaluronate fractions, are relatively easy to manufacture and to obtain governmental regulatory approval on, in comparison to compositions which include two or more significantly dissimilar adhesion/protective components.

In one embodiment, the invention provides a composition defined in claim 13 appended hereto. Preferred embodiments are defined in claims 14 to 19 appended hereto. .

The first hyaluronate fraction preferably has a molecular weight in the range of 350,000 to 800,000, more preferably in the range of 500,000 to 750,000. The second hyaluronate fraction preferably has a molecular weight in the range of 25,000 or 35,000 to 150,000 or about 175,000. The second hyaluronate fraction may have a molecular weight in the range of 30,000 to 100,000. Preferably, the compositions are essentially non-pyrogenic and protein-free.

The ophthalmically acceptable compositions may, and preferably do, include at least one buffer component in an amount effective to control the pH of the composition and/or at least one tonicity adjuster component in an amount effective to control the osmolality of the composition. More preferably, the present compositions include both a buffer component and a tonicity adjuster component. The ophthalmically acceptable compositions of the present invention are preferably sterile.

In another embodiment, the present invention provides use of an aqueous composition defined in claim 1 appended hereto. Preferred embodiments of the use are defined in claims 2 to 12 appended hereto.

Advantageously, the invention allows for removing the natural lens from the eye of a human or animal. These lens removal methods comprise introducing a protective amount of an ophthalmically acceptable composition, such as described herein, into the eye of a human or animal. This introduced composition acts to adhere to and/or protect the eye cell layers and tissues, in particular the corneal endothelium, in proximity to the natural lens in the eye. The natural lens is caused to break into a plurality of particles, e.g., using a conventional surgical procedure which involves a potentially traumatic force associated with a relatively high degree of turbulence. These particles are removed from the eye. The hyaluronate-containing composition is also removed from the eye. In this manner, the defective natural lens, for example, a natural lens which has developed a cataract condition, is effectively removed from the eye without causing undue trauma or adverse effects to the eye cell layers and tissues in proximity to the natural lens which is removed. The presently useful compositions are relatively easy to administer to the eye, provide a very effective and high degree of protection against the potential trauma of the surgical procedure, and are relatively easy to remove, such as by irrigation/aspiration from the eye, after the protection is no longer needed. In particular, the present compositions are maintained in a protective amount on the eye cell layers and tissues in spite of the relative high degree of turbulence associated with causing the natural lens to break into a plurality of particles and/or removing the lens particles from the eye. Without such protection, lens fragments may impact and damage sensitive cell layers and tissues, such as the corneal endothelium.

### Detailed Description of the Invention

Ophthalmically acceptable compositions, in particular in the form of solutions, comprising water, a first alkali and/or alkaline earth metal hyaluronate fraction having a moderate to high molecular weight and a second alkali and/or alkaline earth metal hyaluronate fraction having a relatively low molecular weight, and preferably at least one buffer component and/or at least one tonicity adjuster component, have been found to provide substantial advantages, e.g., in terms of providing adhesion and/or protection to human or animal cell layers and tissues located in the eye which are exposed to trauma, in particular during surgical procedures. The present compositions also have other properties useful in viscoelastic fluids, such as high viscosity at zero shear, elasticity and pseudoplasticity.

The molecular weights noted herein are the weight average molecular weights of the fraction or fractions. This weight average molecular weight can be determined or measured by an indirect method, in particular the limiting viscosity method. The weight average molecular weight may be calculated from the limiting viscosity number by the published equation of Laurent et al, Fractionation of Hyaluronic Acid, Biochimica Et Biophysics Acta, 42, pps 476-485 (1960). Alternately, the weight average molecular weight can be determined using size exclusion chromatography techniques.

The first hyaluronate fractions useful in the present invention have moderate to high molecular weights, in particular, molecular weights in the range of at least 300,000, more particularly in the range of 300,000 to 1,000,000. The first hyaluronate fractions preferably have molecular weights in the range of 350,000 to 800,000, more preferably 500,000 to 750,000.

The presently useful second hyaluronate fractions have molecular weights of less than 200,000, preferably in the range of 25,000 or 35,000 to 150,000 or 175,000. Second hyaluronate fractions having molecular weights in the range of 30,000 to 100,000 are very useful.

As is conventional and well known in the art, the first and second hyaluronate fractions can be obtained from various sources, such as rooster combs and other connective tissue, and from bacterial sources, in particular bacterial fermentation. The molecular weight fractions can be obtained using conventional separation procedures. Alternately, high molecular weight hyaluronates can be converted, for example, hydrolyzed, to lower molecular weight hyaluronates, which are then recovered and used.

The first hyaluronate fraction and second hyaluronate fraction are each preferably independently selected from the group consisting of sodium hyaluronates, potassium hyaluronates, magnesium hyaluronates, calcium hyaluronates and mixtures thereof. More preferably, each of these fractions involves the same metal or metals. Sodium hyaluronate fractions are particularly useful.

The weight ratio of the first hyaluronate fraction to the second hyaluronate fraction in the presently useful compositions is preferably in the range of 0.25 to 4, more preferably 0.5 to 2. The concentration of the first hyaluronate fraction in the presently useful compositions is preferably in the range of 5 mg/ml to 50 mg/ml, more preferably 10 mg/ml or 20 mg/ml to 40 mg/ml.

The present ophthalmically acceptable compositions are preferably essentially free of protein and essentially non-pyrogenic. The present compositions preferably include less than 0.5% by weight of protein based on the total weight of first and second alkali and/or alkaline earth metal hyaluronates. More preferably, the present compositions have no detectable protein content and no detectable pyrogenicity.

The present compositions preferably include a major amount of liquid water, e.g., as a carrier or liquid medium for the hyaluronate fractions. The present ophthalmically acceptable compositions are preferably sterile, in particular prior to being used in the eye.

The present compositions preferably include at least one buffer component in an amount effective to control the pH of the composition and/or at least one tonicity adjuster component in an amount effective to control the osmolality of the composition. More preferably, the present compositions include both a buffer component and a tonicity adjuster component. Such components are useful in maintaining the present compositions ophthalmically acceptable so that, for example, the presence of these compositions in the eye do not result in any undue adverse effect or permanent damage to the eye. The use of one or more tonicity adjuster components is particularly important when the composition is to be applied to one or more portions of the cornea. Such tonicity adjuster components act to enhance the compatibility between the composition and the corneal tissue and/or to avoid damage to the corneal tissue. Such buffer components and tonicity adjuster components may be chosen from those which are conventional and well known in the art. Examples of useful buffer components include, but are not limited to, acetate buffers, citrate buffers, phosphate buffers, borate buffers and mixtures thereof. Phosphate buffers are particularly useful. Useful tonicity adjuster components include, but are not limited to, salts, particularly sodium chloride, potassium chloride, any other suitable ophthalmically acceptable tonicity adjuster component and mixtures thereof. The amounts of buffer component and osmolality control component employed are preferably sufficient to maintain the pH of the compositions in the range of 6 to 8, more preferably 7 to 7.5, and the osmolality of the compositions in the range of 200 to 400, more preferably 250 to 350, mOsmol/kg, respectively.

The present compositions may include one or more other components in amounts effective to provide one or more useful properties and/or benefits to the present compositions.

The present compositions are useful for protecting human or animal ocular or eye cell layers and tissues which are subject to being exposed to trauma, for example, during surgery. In one embodiment, the present method comprises administering a protective amount of such a composition to the ocular or eye cell layers and tissues which are subject to exposure to trauma prior to exposure to the trauma. In this manner, such compositions preferably act to adhere to, or at least partially coat, the cell layers and tissues and/or provide protection for such cell layers and tissues from an imposed trauma.

The present compositions provide particularly good protection in situations where the trauma or potential trauma to which the eye cell layers or tissues is exposed occurs in a dynamic, turbulent environment, such as during a surgical procedure in which a natural lens to be removed is broken into particles, such as by using the conventional phacoemulsification technique.

A specific application in which the present compositions find significant usefulness is in a method for removing the natural lens, e.g., which is diseased, from the eye of a human or animal. In this embodiment, a protective amount of a composition in accordance with the present invention is introduced, such as by being injected, into the eye of a human or animal, for example, through an incision made in the eye. Such compositions can be relatively easily introduced into the eye, for example, using conventional injection cannulas or the like. The composition preferably adheres to at least a portion of the eye cell layers and tissues, in particular the corneal endothelium. in proximity to the natural lens to be removed. With the present composition in place, the natural lens is caused to break into a plurality of particles. One particularly useful approach to breaking up the natural lens is to use a conventional lens emulsification procedure, such as the well known phacoemulsification procedure. Unless the portions of the eye in proximity to the natural lens are provided with protection, the dynamic force used to break up the natural lens and/or the turbulence which often occurs during such lens removal procedures may also damage these other portions of the eye. The use of the present compositions has been found to provide adequate protection for these eye portions, in particular the corneal endothelium, against such potentially traumatic force and/or turbulence. The plurality of lens particles is removed, for example, using a conventional irrigation/aspiration procedure, from the eye. The present hyaluronate-containing composition is also removed, along with the lens particles and/or after further irrigation/aspiration, from the eye. Such removal occurs relatively easily and substantially without detrimentally affecting the remaining portions of the eye.

An intraocular lens can then be implanted in the eye, e.g., using a conventional technique, to replace the removed natural lens. After this implantation, the incision in the eye is closed, such as by suturing. The use of the present compositions, as described above, allows for effective removal of the natural lens from the eye without substantially damaging the eye cell layers and tissues in proximity to the natural lens.

The following non-limiting examples illustrate certain aspects of the present invention.

### EXAMPLES 1 TO 3

A composition (Composition 1) was prepared by mixing two sodium hyaluronate fractions with a standard phosphate buffered saline solution. This composition included 20 mg/ml of a sodium hyaluronate fraction having a weight average molecular weight of 700,000, and 20 mg/ml of a sodium hyaluronate fraction having a weight average molecular weight of 50,000.

Composition 2 (comparative) was a phosphate buffered saline solution containing 30 mg/ml of a sodium hyaluronate fraction having a weight average molecular weight of 500,000, and 40 mg/ml of chondroitin sulfate having a weight average molecular weight of 50,000. Composition 2 is commercially available from Alcon Surgical, Inc., Forth Worth, Texas, and is sold under the trademark Viscoat. Composition 3 (comparative) was a phosphate buffered saline solution containing 10 mg/ml of a sodium hyaluronate fraction having a weight average molecular weight of 3 to 4 million. Composition 3 is commercially available from Pharmacia Ophthalmics, Monrovia, California, and is sold under the trademark Healon.

Each of these compositions was tested as follows. Healthy New Zealand white rabbits were selected for surgery. The surgical procedure performed, using conventional techniques, on the eye of the selected rabbit involved anterior capsulotomy, phacoemulsification of the eye's natural lens (using a standard phacoemulsification unit), irrigation/aspiration and intraocular lens implantation.

Each of the compositions was loaded into a standard BD Hypack syringe. A 25g cannula was attached to each syringe for the injections. Each rabbit received one (1) fluid sample for each eye prior to the procedure. This fluid was replenished as necessary as the surgical procedure progressed.

The surgeon's observations relating to these compositions included the following:
Composition 1 was difficult to inject through a 25g cannula. It maintained the anterior chamber well and adhered strongly to the corneal endothelium cells during the phacoemulsification procedure, but was difficult to remove during irrigation/aspiration due to its relatively high viscosity and stickiness.
Composition 2 was somewhat easier to inject than Composition 1 and it maintained the chamber approximately the same as Composition 1. Composition 2 coated the corneal endothelium well and was somewhat difficult to remove by irrigation/aspiration.
Composition 3 was very easy to inject. However, it did not appear to maintain the anterior chamber as well as Compositions 1 and 2 during the anterior capsulotomy procedure. Furthermore, Composition 3 flushed completely out of the eye upon initiation of phacoemulsification and did not appear to coat either the endothelial cells or the intraocular lens well. Composition 3 was the most readily removed during irrigation/aspiration.

These results indicate that Composition 1 provided the desired protection during the phacoemulsification procedure. Such protection was as good as or better than the protection achieved using the other compositions tested. Also, the relatively high viscosity and stickiness of Composition 1 can be reduced by reducing the concentrations of one or both of the sodium hyaluronate components and/or by adjusting the ratio of the different molecular weight sodium hyaluronate components and/or by adjusting the molecular weight of one or both of the sodium hyaluronate components.

### EXAMPLES 4 TO 8

A composition (Composition 4) was prepared by mixing two sodium hyaluronate fractions with a standard saline solution. This composition included 30 mg/ml of a sodium hyaluronate fraction having a weight average molecular weight of 700,000, and 5 mg/ml of a sodium hyaluronate fraction having a weight average molecular weight of 120,000. The viscosity of Composition 4 was 75 Pas (75,000 centipoises) at a shear rate of 1 sec⁻¹.

A composition (Composition 5) was prepared by mixing two sodium hyaluronate fractions with a standard saline solution. This composition included 25 mg/ml of a sodium hyaluronate fraction having a weight average molecular weight of 700,000, and 10 mg/ml of a sodium hyaluronate fraction having a weight average molecular weight of 120,000. The viscosity of Composition 5 was 45 Pas (45,000 centipoises) at a shear rate of 1 sec-1.

A composition (Composition 6) was prepared by mixing two sodium hyaluronate fractions with a standard saline solution. This composition included 20 mg/ml of a sodium hyaluronate fraction having a weight average molecular weight of 700,000, and 30 mg/ml of a sodium hyaluronate fraction having a weight average molecular weight of 120,000. The viscosity of Composition 6 was 20 Pas (20,000 centipoises) at a shear rate of 1 sec⁻¹.

Composition 7 (comparative) was similar to Composition 2. The viscosity of Composition 7 was 45 Pas (45,000 centipoises) at a shear rate of 1 sec⁻¹.

Composition 8 (comparative) was similar to Composition 3. The viscosity of Composition 8 was 45 Pas (45,000 centipoises) at a shear rate of 1 sec⁻¹.

Each of these compositions was tested as follows. Healthy New Zealand white rabbits were selected for surgery. The surgical procedure performed, using conventional techniques, on the eye of the selected rabbit involved anterior capsulorhexis, phacoemulsification of the eye's natural lens (using a standard phacoemulsification unit), irrigation/aspiration and intraocular lens implantation. Each of the compositions was loaded into a 3 cc BD syringe. A 27 g cannula was attached to each syringe for the injections. Each rabbit received one (1) fluid sample for each eye prior to the procedure. This fluid was replenished as necessary as the surgical procedure progressed.

The surgeon's observations relating to these compositions included the following:
Composition 4 injected through a 27 g cannula with some difficulty. It maintained the anterior chamber well during capsulorhexis, and adhered well to the corneal endothelium during the phacoemulsification procedure. This material was removed fairly easily during irrigation/aspiration.
Composition 5 injected through a 27 g cannula with some difficulty. It did not maintain the anterior chamber during capsulorhexis as well as Composition 4. Composition 5 adhered well to the corneal endothelium during the phacoemulsification procedure and was removed easily during irrigation/aspiration.
Composition 6 was difficult to inject through a 27 g cannula. It maintained the anterior chamber during capsulorhexis approximately the same as Composition 4. Composition 6 adhered well to the corneal endothelium during the phacoemulsification procedure, and was easily removed during irrigation/aspiration.
Composition 7 was somewhat easier to inject than Compositions 4 and 5, and was much easier to inject than Composition 6. However, Composition 7 did not maintain the anterior chamber during capsulorhexis as well as either Composition 4 or Composition 6. It maintained the anterior chamber approximately the same as Composition 5, and adhered about as well to the corneal endothelium during the phacoemulsification procedure as did either Composition 5 or Composition 6. Composition 7 was removed easily during irrigation/aspiration.
Composition 8 was very easy to inject. However, it did not maintain the anterior chamber during capsulorhexis as well as any of the other compositions. Composition 8 completely flushed out of the eye upon initiation of phacoemulsification and did not appear to coat the corneal endothelium well. Composition 8 was the most readily removed by irrigation/aspiration.

These results indicate that Compositions 4, 5 and 6, in accordance with the present invention, provided superior protection during capsulorhexis and the phacoemulsification procedure relative to Composition 8 which includes a high molecular weight sodium hyaluronate fraction. In addition, the present compositions, that is Compositions 4, 5 and 6, provide substantially as good or better protection than did Composition 7, which includes both a sodium hyaluronate fraction and a chondroitin sulfate fraction. Of course, the present compositions have advantages, for example, in ease of manufacture, relative to Composition 7 which includes two significantly different materials, sodium hyaluronate and chondroitin sulfate.

When comparing Compositions 1, 4, 5 and 6 (in accordance with the present invention) with Compositions 3 and 8, the present compositions provide results which are generally superior with respect to compositions containing a single high molecular weight sodium hyaluronate fraction. These results also demonstrate that compositions including a first hyaluronate fraction having a weight average molecular weight in the range of 500,000 to 750,000 and a second hyaluronate fraction having a weight average molecular weight in the range of 25,000 or 35,000 to 150,000 or about 175,000 provide substantially comparable results. For example, changing the weight average molecular weight of the second hyaluronate fraction from 50,000 (Composition 1) to 120,000 (Compositions 4, 5 and 6) did not result in any substantial change in the beneficial properties achieved using the present compositions. The first hyaluronate fraction tested, that is having a weight average molecular weight of 700,000 is representative of hyaluronates having weight average molecular weights in the range of 500,000 to 750,000. That is, no substantial difference in composition performance would occur by changing the weight average molecular weight of the first hyaluronate fraction within the range 500,000 and 750,000.

In other words, the data presented in these Examples is representative of compositions including a first hyaluronate fraction having high molecular weight average molecular weight in the range of 500,000 to 750,000 and a second hyaluronate fraction having a weight average molecular weight in the range of 25,000 or 35,000 to 150,000 to 175,000. These Examples make clear that the present compositions provide as good or better performance than the current commercially available materials, as set forth in Compositions 2, 3, 7 and 8. In particular, the present compositions provide improved performance relative to compositions which include only a single high molecular weight hyaluronate fraction, that is Compositions 4 and 8. This result is clearly unexpected and provides substantial benefits, for example, in terms of improved performance while being relatively easy to manufacture and to control the quality of, and to obtain governmental regulatory approval on.

While this invention has been described with respect to various specific examples and embodiments, it is to be understood that the invention is not limited thereto and that it can be variously practiced within the scope of the following claims.

## Claims

1. The use of an aqueous composition consisting of a first metal hyaluronate fraction having a weight average molecular weight in the range of 500,000 to 750,000, and a second metal hyaluronate fraction having a weight average molecular weight in the range of 25,000 to 175,000, the first and second metal hyaluronate fractions each being independently selected from alkali metal hyaluronates, alkaline earth metal hyaluronates and mixtures thereof; for the manufacture of a medicament for protecting human or animal ocular tissue against surgical trauma.

2. The use according to Claim 1 wherein the composition additionally consists of at least one buffer component in an amount effective to control the pH of the composition and at least one tonicity adjuster component in an amount effective to control the osmolality of the composition.

3. The use according to Claim 2 wherein the composition has a pH in the range of 6 to 8, and an osmolality in the range of 200 to 400 mOsmol/kg, and the second metal hyaluronate fraction has a weight average molecular weight in the range of 35,000 to 150,000.

4. The use according to any preceding Claim wherein the second metal hyaluronate fraction has a weight average molecular weight in the range of 30,000 to 100,000.

5. The use according to any preceding Claim wherein the first metal hyaluronate fraction and the second metal hyaluronate fraction are each independently selected from the group consisting of sodium hyaluronates, potassium hyaluronates, magnesium hyaluronates, calcium hyaluronates and mixtures thereof.

6. The use according to any preceding Claim wherein the first metal hyaluronate fraction is a first sodium hyaluronate fraction and a second metal hyaluronate fraction is a second sodium hyaluronate fraction.

7. The use according to any preceding Claim wherein the weight ratio of the first metal hyaluronate fraction to the second metal hyaluronate fraction in the composition is in the range of 0.25 to 4, the first metal hyaluronate fraction being present in the composition in a concentration in the range of 5 mg/ml to 50 mg/ml.

8. The use according to any preceding Claim wherein the composition is sterile and is essentially non-pyrogenic.

9. The use according to Claim 6 wherein the medicament is for use in protecting human or animal ocular tissue against surgical trauma during removal of the natural lens from the eye of a human or animal in which the natural lens is broken into a plurality of particles and the plurality of particles are then removed from the eye.

10. The use according to Claim 9 wherein the composition includes at least one buffer component in an amount effective to control the pH of the composition and at least one tonicity adjuster component in an amount effective to control the osmolality of the composition.

11. The use according to Claim 10 wherein the composition has a pH in the range of 7 to 7.5, and an osmolality in the range of 250 to 350 mOsmol/kg, and the second sodium hyaluronate fraction has a weight average molecular weight in the range of 35,000 to 150,000.

12. The use according to Claim 9 wherein the weight ratio of the first sodium hyaluronate fraction to the second sodium hyaluronate fraction in the composition is in the range of 0.25 to 4.

13. A composition comprising water, a first metal hyaluronate fraction having a weight average molecular weight in the range of 500,000 to 750,000, a second metal hyaluronate fraction having a weight average molecular weight in the range of 25,000 to 175,000, at least one buffer component in an amount effective to control the pH of said composition and at least one tonicity adjuster component in an amount effective to control the osmolality of said composition, said composition being ophthalmically acceptable, said first metal hyaluronate fraction and said second metal hyaluronate fraction each being present in said composition in an intraocularly effective amount to protect ocular tissue against surgical trauma, and said first metal hyaluronate fraction and said second metal hyaluronate fraction each being independently selected from the group consisting of alkali metal hyaluronates, alkaline earth metal hyaluronates and mixtures thereof.

14. The composition of Claim 13 wherein said composition has a pH in the range of 6 to 8, and an osmolality in the range of 200 to 400 mOsmol/kg, and said second metal hyaluronate fraction has a weight average molecular weight in the range of 35,000 to 150,000, said composition being sterile and essentially non-pyrogenic.

15. The composition of Claim 13 wherein said second metal hyaluronate fraction has a molecular weight in the range of 30,000 to 100,000.

16. The composition of Claim 13 wherein said first metal hyaluronate fraction and said second metal hyaluronate fraction are each independently selected from the group consisting of sodium hyaluronates, potassium hyaluronates, magnesium hyaluronates, calcium hyaluronates and mixtures thereof.

17. The composition of Claim 13 wherein said first metal hyaluronate fraction is a first sodium hyaluronate fraction and said second metal hyaluronate fraction is a second sodium hyaluronate fraction.

18. The composition of Claim 13 wherein the weight ratio of said first metal hyaluronate fraction to said second metal hyaluronate fraction in said composition is in the range of 0.25 to 4,

19. The composition of Claim 18 wherein said first metal hyaluronate fraction is present in said composition in a concentration in the range of 5 mg/ml to 50 mg/ml.

## Patentansprüche

1. Verwendung einer wässrigen Zusammensetzung, die aus einer ersten Metall-Hyaluronat-Fraktion mit einem gewichteten mittleren Molekulargewicht im Bereich von 500000 bis 750000 und aus einer zweiten Metall-Hyaluronat-Fraktion mit einem gewichteten mittleren Molekulargewicht in dem Bereich von 25000 bis 175000 besteht, wobei die ersten und zweiten Metall-Hyaluronat-Fraktionen jeweils unabhängig aus Alkalimetall-Hyaluronaten, Erdalkalimetall-Hyaluronaten und Mischungen hiervon ausgewählt sind; für die Herstellung eines Medikaments zum Schutz menschlichen oder tierischen Augengewebes gegen chirurgisches Trauma.

2. Verwendung nach Anspruch 1, bei der die Zusammensetzung zusätzlich aus einer Pufferkomponente in einer Menge, wirksam ist, um den pH-Wart der Zusammensetzung zu kontrollieren, und aus einer Tonizitäts-Einstellkomponente in einer Menge besteht, die wirksam ist, um die Osmolalität der Zusammensetzung zu kontrollieren.

3. Verwendung nach Anspruch 2, bei der die Zusammensetzung einen pH-Wert in dem Bereich von 6 bis 8 und eine Osmolalität in dem Bereich von 200 bis 400 mOsmol/kg aufweist, und bei der die zweite Metall-Hyaluronat-Fraktion ein gewichtetes mittleres Molekulargewicht in dem Bereich von 35000 bis 150000 aufweist.

4. Verwendung nach einem der vorhergehenden Ansprüche, bei der die zweite Metall-Hyaluronat-Fraktion ein gewichtetes mittleres Molekulargewicht in dem Bereich von 30000 bis 100000 aufweist.

5. Verwendung nach einem der vorhergehenden Ansprüche, bei der die erste Metall-Hyaluronat-Fraktion und die zweite Metall-Hyaluronat-Fraktion jeweils unabhängig aus der Gruppe ausgewählt sind, die aus Natrium-Hyaluronaten, Kalium-Hyaluronaten, Magnesium-Hyaluronaten, Calzium-Hyaluronaten und Mischungen hiervon besteht.

6. Verwendung nach einem der vorhergehenden Ansprüche, bei der die erste Metall-Hyaluronat-Fraktion eine erste Natrium-Hyaluronat-Fraktion ist und eine zweite Metall-Hyaluronat-Fraktion eine zweite Natrium-Hyaluronat-Fraktion ist.

7. Verwendung nach einem der vorhergehenden Ansprüche, bei der das Gewichtsverhältnis der ersten Metall-Hyaluronat-Fraktion zu der zweiten Metall-Hyaluronat-Fraktion in der Zusammensetzung in dem Bereich von 0,25 bis 4 liegt, wobei die erste Metall-Hyaluronat-Fraktion in der Zusammensetzung in einer Konzentration in dem Bereich von 5 mg/ml bis 50 mg/ml vorliegt.

8. Verwendung nach einem der vorhergehenden Ansprüche, bei der die Zusammensetzung steril und im wesentlichen nicht-pyrogen ist.

9. Verwendung nach Anspruch 6, bei der das Medikament zur Verwendung beim Schutz von menschlichem oder tierischem Augengewebe gegen chirurgisches Trauma während der Entfernung der natürlichen Linse aus dem Auge eines Menschen oder Tieres dient, bei der die natürliche Linse in eine Vielzahl von Teilchen aufgebrochen und die Vielzahl der Teilchen dann aus dem Auge entfernt wird.

10. Verwendung nach Anspruch 9, bei der die Zusammensetzung zumindest eine Pufferkomponente in einer Menge, die zur Kontrolle des pH-Wertes der Zusammensetzung wirksam ist, und zumindest eine Tonizitäts-Einstellkomponente in einer Menge einschließt, die zur Kontrolle der Osmolalität der Zusammensetzung wirksam ist.

11. Verwendung nach Anspruch 10, bei der die Zusammensetzung einen pH-Wert in dem Bereich von 7 bis 7,5 und eine Osmolalität in dem Bereich von 250 bis 350 mOsmol/kg aufweist, und bei der die zweite Natrium-Hyaluronat-Fraktion ein gewichtetes mittleres Molekulargewicht in dem Bereich von 35000 bis 150000 aufweist.

12. Verwendung nach Anspruch 9, bei der das Gewichtsverhältnis der ersten Natrium-Hyaluronat-Fraktion zu der zweiten Natrium-Hyaluronat-Fraktion in der Zusammensetzung in dem Bereich von 0,25 bis 4 liegt.

13. Zusammensetzung, die Wasser, eine erste Metall-Hyaiuronat-Fraktion mit einem gewichteten mittleren Molekulargewicht in dem Bereich von 500000 bis 750000, eine zweite Metall-Hyaluronal-Praktion mit einem gewichteten mittleren Molekulargewicht in dem Bereich von 25000 bis 175000, zumindesl eine Pufferkomponente in eine Menge, die zur Kontrolle des pH-Wertes der Zusammensetzung wirksam ist, und zumindest eine Tonizitäts-Einstellkomponente in einer Menge umfaßt, die zur Kontrolle der Osmolalität der Zusammensetzung wirksam ist, wobei die Zusammensetzung augenverträglich ist, wobei die erste Metall-Hyaluronat-Fraktion und die zweite Metall-Hyaluronat-Fraktion jeweils in der Zusammensetzung in einer intraokular wirksamen Menge vorliegen, um Augengewebe gegen ein chirurgisches Trauma zu schützen, und wobei die erste Metall-Hyaluronat-Fraktion und die zweite Metall-Hyaluronat-Fraktion jeweils unabhängig aus der Gruppe ausgewählt sind, die aus Atkalimetall-Hyaluronaten, Erdalkalimetall-Hyaluronaten und Mischungen hiervon besteht.

14. Zusammensetzung nach Anspruch 13, bei der die Zusammensetzung einen pH-Wert in dem Bereich von 6 bis 8 und eine Osmolalität in dem Bereich von 200 bis 400 mOsmol/kg aufweist, und bei der die zweite Natrium-Hyaluronat-Fraktion ein gewichtetes mittleres Molekulargewicht in dem Bereich von 35000 bis 150000 aufweist, wobei die Zusammensetzung steril und im wesentlichen nicht-pyrogen ist.

15. Zusammensetzung nach Anspruch 13, bei der bei der die zweite Metall-Hyaluronat-Fraktion ein Molekulargewicht in dem Bereich von 30000 bis 100000 aufweist.

16. Zusammensetzung nach Anspruch 13, bei der die erste Metall-Hyaluronat-Fraktion und die zweite Metall-Hyaluronat-Fraktion jeweils unabhängig aus der Gruppe ausgewählt sind, die aus Natrium-Hyaluronaten, Kalium-Hyaluronaten, Magnesium-Hyaluronaten, Calzium-Hyaluronaten und Mischungen hiervon besteht.

17. Zusammensetzung nach Anspruch 13, bei der die erste Metatl-Hyaluronat-Fraktion eine erste Natrium-Hyaluronat-Fraktion ist und die zweite Metall-Hyaluronat-Fraktion eine zweite Natrium-Hyaluronat-Fraktion ist.

18. Zusammensetzung nach Anspruch 13, bei der das Gewichtsverhältnis der ersten Metall-Hyaluronat-Fraktion zu der zweiten Metall- Hyaluronat-Fraktion in der Zusammensetzung in dem Bereich von 0,25 bis 4 liegt.

19. Zusammensetzung nach Anspruch 18, bei der die erste Metall-Hyaluronat-Fraktion in der Zusammensetzung in einer Konzentration in dem Bereich von 5 mg/ml bis 50 mg/ml vorliegt.

## Revendications

1. Utilisation d'une composition aqueuse consistant en une première fraction de hyaluronate de métal ayant un poids moléculaire moyen en poids dans l'intervalle de 500000 à 750000 et une seconde fraction d'hyaluronate de métal ayant un poids moléculaire moyen en poids dans l'intervalle de 25000 à 175000, les première et seconde fractions d'hyaluronates de métal étant chacune indépendamment choisies parmi les hyaluronates de métal alcalin, hyaluronates de métal alcalino-terreux et des mélanges de ceux-ci; pour la fabrication d'un médicament pour protéger le tissu oculaire humain ou animal contre un trauma chirurgical.

2. Utilisation selon la revendication 1 où la composition consiste en outre, en au moins un composé tampon en une quantité efficace pour régler le pH de la composition et au moins un composant ajusteur de la tonicité en une quantité efficace pour régler l'osmolalité de la composition.

3. Utilisation selon la revendication 2 où la composition a un pH dans l'intervalle de 6 à 8, et une osmolalité dans l'intervalle de 200 à 400 mOsmol/kg, et la seconde fraction d'hyaluronate de métal a un poids moléculaire moyen en poids dans l'intervalle de 35000 à 150000.

4. Utilisation selon l'une quelconque des revendications précédentes où la seconde fraction d'hyaluronate a un poids moléculaire moyen dans l'intervalle de 30000 à 100000.

5. Utilisation selon l'une quelconque des revendications précédentes où la première fraction d'hyaluronate de métal et la seconde fraction d'hyaluronate de métal sont chacune indépendamment choisies dans le groupe consistant en hyaluronate de sodium, hyaluronate de potassium, hyaluronate de magnésium, hyaluronate de calcium et mélanges de ceux-ci.

6. Utilisation selon l'une quelconque des revendications précédentes où la première fraction d'hyaluronate de métal est une première fraction d'hyaluronate de sodium et la seconde fraction d'hyaluronate de métal est une seconde fraction d'hyaluronate de sodium.

7. Utilisation selon l'une quelconque des revendications précédentes où le rapport pondéral de la première fraction d'hyaluronate de métal à la seconde fraction d'hyaluronate de métal dans la composition est dans l'intervalle de 0,25 à 4, la première fraction d'hyaluronate de métal étant présente dans la composition en une fraction dans l'intervalle de 5 mg/ml à 50 mg/ml.

8. Utilisation selon l'une quelconque des revendications précédentes où la composition est stérile et est essentiellement non-pyrogène.

9. Utilisation selon la revendication 6 où le médicament sert à être utilisé pour protéger le tissu oculaire humain ou animal contre un trauma chirurgical pendant l'enlèvement du cristallin de l'oeil d'un animal où le cristallin est rompu en une pluralité de particules et la pluralité des particules est ensuite enlevée de l'oeil.

10. Utilisation selon la revendication 9 où la composition comprend au moins un composé tampon en une quantité efficace pour régler le pH de la composition et au moins un composant ajusteur de la tonicité en une quantité efficace pour régler l'osmolalité de la composition.

11. Utilisation selon la revendication 10 où la composition a un pH dans l'intervalle de 7 à 7,5, et une osmolalité dans l'intervalle de 250 à 350 mOsmol/kg, et la seconde fraction d'hyaluronate de sodium a un poids moléculaire moyen en poids dans l'intervalle de 35000 à 150000.

12. Utilisation selon la revendication 9 où le rapport pondéral de la première fraction d'hyaluronate de sodium à la seconde fraction d'hyaluronate de sodium dans la composition et dans l'intervalle de 0,25 à 4.

13. Composition comprenant de l'eau, une première fraction de hyaluronate de métal ayant un poids moléculaire moyen en poids dans l'intervalle de 500000 à 750000 et une seconde fraction d'hyaluronate de métal ayant un poids moléculaire moyen en poids dans l'intervalle de 25000 à 175000, au moins un composant tampon en une quantité efficace pour régler le pH de cette composition et au moins un composant ajusteur de la tonicité en une quantité efficace pour régler l'osmolalité de la composition, cette composition étant ophtalmiquement acceptable, cette première fraction d'hyaluronate de métal et cette seconde fraction d'hyaluronate de métal étant chacune présente dans cette composition en une quantité intraoculairement efficace pour protéger le tissu oculaire humain ou animal contre un trauma chirurgical, et cette première fraction d'hyaluronate de métal et cette seconde fraction d'hyaluronate de métal étant chacune indépendamment choisies dans le groupe consistant en hyaluronate de métal alcalin, hyaluronate de métal alcalino-terreux et des mélanges de ceux-ci.

14. Composition selon la revendication 13 où cette composition a un pH dans l'intervalle de 6 à 8, et une osmolalité dans l'intervalle de 200 à 400 mOsmol/kg, et cette seconde fraction d'hyaluronate de métal a un poids moléculaire moyen en poids dans l'intervalle de 35000 à 150000, cette composition étant stérile et essentiellement non-pyrogène.

15. Composition selon la revendication 13 où cette seconde fraction d'hyaluronate de métal a un poids moléculaire dans l'intervalle de 30000 à 100000.

16. Composition selon la revendication 13 où cette première fraction d'hyaluronate de métal et cette seconde fraction d'hyaluronate de métal sont chacune indépendamment choisies dans le groupe consistant en hyaluronate de sodium, hyaluronate de potassium, hyaluronate de magnésium, hyaluronate de calcium et mélanges de ceux-ci.

17. Composition selon la revendication 13 où cette première fraction d'hyaluronate de métal est une première fraction d'hyaluronate de sodium et cette seconde fraction d'hyaluronate de métal est une seconde fraction d'hyaluronate de sodium.

18. Composition selon la revendication 13 où le rapport pondéral de cette première fraction d'hyaluronate de métal à cette seconde fraction d'hyaluronate de métal dans cette composition est dans l'intervalle de 0,25 à 4.

19. Composition de la revendication 18 où cette première fraction d'hyaluronate de métal est présente dans cette composition en une concentration dans l'intervalle de 5 mg/ml à 50 mg/ml.
